# EUROPÄISCHE PATENTSCHRIFT

(11) **EP 1 002 899 B1**
(45) Veröffentlichungstag und Bekanntmachung des Hinweises auf die Patenterteilung: **12.04.2006**
(21) Anmeldenummer: 99122483.3
(22) Anmeldetag: 11.11.1999
(51) Int. Cl.: D21H 25/18

(54) **Überkritisches Kohlendioxid zur antimikrobiellen Behandlung kontaminierter Papiere**
Super critical carbon dioxide for the antimicrobial treatment of contaminated paper
Dioxyde de carbone supercritique pour le traitement antimicrobien de papiers contaminés

(30) Priorität: 11.11.1998 DE 19852070
(43) Veröffentlichungstag der Anmeldung: 24.05.2000
(73) Patentinhaber: ZFB Zentrum für Bucherhaltung GmbH, 04329 Leipzig (DE)
(72) Erfinder: Anders, Manfred, 70180 Stuttgart (DE)
(74) Vertreter: Alber, Norbert

(56) Entgegenhaltungen:
- DE-C- 19 607 053
- US-A- 4 051 276
- US-A- 4 522 843
- US-A- 4 619 735

## Beschreibung

Die Erfindung betrifft die Erhaltung kultureller Güter, insbesondere Papiere, wie etwa Bücher, aber auch andere kultureller Gegenstände aus Holz, Textilien etc.. Alle aus organischen Materialien bestehenden Güter unterliegen einer mehr oder minder starken Zersetzung durch Mikroorganismen. Dadurch werden diese Materialien (oft handelt es sich um wertvolle Kulturgüter) irreversibel beschädigt oder gar zerstört.

Mikrobielle Kontaminationen an Papieren werden durch verschiedene Pilze und Bakterien verursacht. Neben den Hauptbestandteilen des Papiers, der Zellulose, werden auch Hilfsstoffe, die bei der Herstellung des Papiers und des Buches verwendet werden, durch Mikroorganismen angegriffen und zersetzt. Dieser Vorgang ist verbunden mit dem Papierabbau, der Ausbreitung der Mikroorganismen und der Akkumulation von Stoffwechselprodukten. Beim Zelluloseabbau entstehen Zuckermoleküle, deren mikrobielle Umsetzung in vielen Fällen mit Säurebildung verbunden ist. Das bedeutet, daß mikrobielle Kontaminanten nicht nur zum aktiven Abbau der Papiermatrix beitragen, sondern die Versäuerung des Dokuments weiter beschleunigen.

Ein Teilziel für die Erhaltung und Konservierung dieser kulturellen Güter besteht daher in der Inaktivierung solcher mikrobieller Kontaminanten. Die Konservierung hat zum Ziel, das Material über einen genügend langen Zeitraum bei den vorhandenen Bedingungen unverändert zu erhalten. Zu diesem Zweck müssen alle schädigenden Einflüsse ausgeschaltet werden. Die große Gefahr, die von Mikroorganismen ausgeht, liegt einmal in ihrer raschen Vermehrung und zum anderen in ihren intensiven Stoffwechselleistungen. In diesem Zusammenhang sind ganz unterschiedliche Methoden der Inaktivierung der Mikroorganismen bekannt, nämlich entweder auf chemischem Wege, oder auf physikalischem Wege.

Mit chemischen Mitteln erfolgt die Zerstörung der Mikroorganismen meist durch hochgiftige Stoffe wie etwa Ethylenoxid.

Der Nachteil der Behandlung mit einem Giftstoff wie Ethylenoxid besteht darin, daß diese, z. T. karzinogenen und mutagenen, Substanzen, von dem zu behandelnden Objekt absorbiert werden und danach langsam wieder an die Umgebung abgegeben werden.

Damit besteht eine starke Gefährdung für die Nutzer derartiger Gegenstände, etwa mikrobiell kontaminierter Papiere, besonders wenn in einem Raum eine größere Ansammlung davon vorhanden ist, wie etwa in einer Bibliothek.

Es wurden auch farbliche Veränderungen an einigen Büchern nach einer Behandlung mit Ethylenoxid gefunden.

Als physikalische Behandlungsmethode wird momentan meist die radioaktive Bestrahlung, in der Regel mit Kobalt 60, eingesetzt. Der Nachteil besteht in einer z. T. starken Schädigung des Materials, die sich in einer beschleunigten Alterung bzw. Versprödung des Materials äußert.

Darüber hinaus gibt es weitere Verfahren zur Inaktivierung mikrobiozider Kontaminanten, die die vorgenannten Nachteile zwar zum Teil vermeiden, jedoch auf der anderen Seite nicht als Massenbehandlungmethode eingesetzt werden können, sondern lediglich die Behandlung kleiner Mengen, beispielsweise in Form einzelner Blätter, gestatten.

Der schädigende Effekt gerade bei Schriftgut und Büchern liegt beispielsweise oftmals in einem Verblassen oder Ausbluten der dort verwendeten Tinten. Ein Verblassen kann durch chemische Reaktion mit den Inhaltsstoffen der Tinte erfolgen, ein Ausbluten oder Verlaufen der Tinten durch Nachreicherung des Lösungsmittels der Tinte und damit Anlösung der Tinte, und das Bewirken von Fließvorgängen innerhalb des Papiers aufgrund kapillarer Saugwirkungen.

***Die Behandlung von Papieren bzw. Büchern mit überkritischem Kohlendioxid zur Zerstörung der darin enthaltenen Mikroorganismen ist prinzipiell bereits aus der US-A-4619735 bekannt.***

Es ist daher die Aufgabe der vorliegenden Erfindung, ein Massenbehandlungsverfahren zur Bekämpfung von Mikroorganismen in organischen Gegenständen, insbesondere Papieren in Form von ganzen Büchern, zur Verfügung zu stellen, welches weder giftige Substanzen im Objekt zurückläßt noch für das Objekt beschädigende Effekte mit sich bringt.

Ziel der Behandlung ist es die im Behandlungsgut vorhandenen Mikroorganismen (auch Insekten) soweit zu inaktivieren, daß sie nicht mehr das Material schädigen können. D.h. Hemmung und vorhandene Keime möglichst durch Abtötung bzw. Inaktivierung der Mikroorganismen, wobei die nicht mehr vermehrungsfähigen Formen im Behandlungsgut bleiben. Unter Umständen kann durch Zusatz eines chemischen Konservierungsmittels das Material vor einem Befall durch neue Mikroorganismen geschützt werden.

Diese Aufgabe wird durch die Merkmale des Anspruchs 1 gelöst. Vorteilhafte Ausführungsform ergeben sich aus den Unteransprüchen.

Der überkritische Zustand einer Substanz wird meist auch als sogenannter vierter Aggregatzustand bezeichnet.

Erwärmt man in einem abgeschlossenen Volumen, das zum Teil mit einer Flüssigkeit gefüllt ist, eine Flüssigkeit (die im Falle von Kohlendioxid auch aus einem komprimierten Gas bestehen kann), so wandern mit steigender Temperatur immer mehr Flüssigkeitsmoleküle in den Raum über der Flüssigkeit, die Dichte des Dampfes nimmt zu, während die Dichte der Flüssigkeit sinkt. Bei weiterem Erhitzen wird schließlich ein Punkt erreicht, an dem die Dichten von Flüssigkeit und Dampf gleich sind, so daß zwischen Flüssigkeit und Dampf kein Unterschied mehr besteht und die Phasengrenze verschwindet.

Dieser Vorgang läuft jedoch nur oberhalb bestimmter kritischer Werte der Zustandsgrößen Druck und Temperatur ab. Man bezeichnet diese Temperatur als kritische Temperatur, den dazugehörigen Druck als den kritischen Druck. Stoffe im Gebiet oberhalb der durch die kritischen Daten gegebenen Grenzen befinden sich im überkritischen Zustand.

Ein Vergleich der physikalisch-chemischen Eigenschaften von überkritischen, fluiden Lösungsmitteln mit den Eigenschaften von Flüssigkeiten und Gasen zeigt, daß die überkritische Phase bezüglich der Dichte und dem Lösungsvermögen von anderen Stoffen mehr einer Flüssigkeit, dagegen in der Viskosität (und damit im Strömungsverhalten und im Diffusionskoeffizient) mehr einem Gas gleicht.

Da die Lösefähigkeit überkritischer Fluide von deren Dichte und Temperatur abhängt, kann durch die Wahl dieser Parameter die Lösefähigkeit gezielt variiert werden. Aus niedrigen Drücken resultieren geringe Dichten und somit eine geringe Lösefähigkeit. Umgekehrt bedingen hohe Drücke hohe Dichten und eine große Lösefähigkeit.

Neben der Lösefähigkeit beeinflußt die Temperatur den Diffusionskoeffizienten. Eine Temperaturerhöhung bewirkt auch eine Erhöhung der Diffusionsgeschwindigkeit. Die Polarität von überkritischen Kohlendioxid gleicht einem unpolaren Lösungsmittel. Es konnten bisher keine schädigende Wirkung auf das behandelte Schriftgut durch überkritisches Kohlendioxid gefunden werden.

Kohlendioxid geht in den Zustand als überkritisches Kohlendioxid (ÜK-CO₂) über bei einem Druck von mindestens 72,8 Atmosphären und einer Temperatur von mindestens 31,1°C. Während in der Vergangenheit die Veränderung der physikalischen Lösungsfähigkeit des überkritischen Zustands im Vordergrund stand, in dem durch Eintritt in den überkritischen Zustand ein gewünschter Stoff in hohem Maße im Lösungsmittel gelöst, und bei Unterschreiten der überkritischen Grenzen durch Nachlassen der Lösungsfähigkeit am oder im Objekt ausgefällt wurde, steht bei der vorliegenden Erfindung hauptsächlich die festgestellte mikrobielle Wirkung des ÜK-CO₂ im Vordergrund.

Im folgenden werden beispielhaft ein Behandlungsverfahren für historische Papiere und Schriften, insbesondere in Form ganzer Bücher, beschrieben.

Dabei werden diese Bücher weder aufgefächert noch in anderer Art und Weise auf mechanischem Wege die Zugänglichkeit der beaufschlagenden Substanzen verbessert. Damit ist es möglich, beispielsweise bei Behandlung in einem Autoklaven, diesen ohne Einbeziehung von nennenswerten Zwischenräumen, Strömungskanälen oder ähnlichem soweit als möglich vollständig mit Büchern und Schriften oder anderen Gütern zu befüllen und damit mit geringem Zeit- und Arbeitsaufwand eine Massenbehandlung durchzuführen.

Dabei spielt unter anderem die Aktivität bzw. Lebensform der in den Büchern bzw. Papieren enthaltenen, schädigenden Mikroorganismen eine Rolle, die stark vom Feuchtigkeitsgehalt und den vorhergehenden Lagerbedingungen des mikrobiell befallenen Schriftguts abhängt.

Einige Mikroorganismen, wie etwa Pilze, können bei Trockenheit einen Oberdauerungszustand (latente Phase), etwa in Form von Sporen, eingehen, welche über sehr lange Zeiträume lebensfähig bleiben, und auch Inaktivierungsversuchen wesentlich resistenter gegenüberstehen als andere Lebensformen von Mikroorganismen. Mikroorganismen sind hingegen in ihrer aktiven Wachstumsphase wesentlich empfindlicher. Die Behandlungsart und auch -dauer unterscheidet sich damit je nach Ausgangszustand bzw. Vorbehandlung in den Objekten enthaltenen Mikroorganismen und damit den Zustand des Objektes selbst.

Ein weiterer wesentlicher Aspekt besteht darin, daß durch die beaufschlagende Substanz innerhalb der Papiere keine polare Flüssigkeit erzeugt oder abgelagert und dabei oder anschließend einem Fließvorgang unterworfen werden darf, da dies innerhalb der Schriften zu einem irreversiblen Verlaufen (Ausbluten) der Tinten führen kann.

In- und Output von Behandlungssubstanzen oder Inhaltsstoffen des Schriftguts, egal ob mittels des beaufschlagenden überkritischen Kohlendioxid oder anderer Substanzen während einer Vorkonditionierung, sollen somit nur im gasförmigen, nicht jedoch im flüssigen Zustand vonstatten gehen.

Insbesondere ist es sehr empfehlenswert, jegliche Strömungsvorgänge - nicht nur von Flüssigkeiten, sondern auch von Gasen, die Feuchtigkeit in gasförmigem Zustand gelöst enthalten, jedoch am Objekt kondensieren könnten - zu vermeiden.

Daher sollte insbesondere bei der Behandlung im Autoklaven eine Strömung innerhalb des Autoklaven soweit wie möglich vermieden werden. Ein Umpumpen der beaufschlagenden Substanzen, etwa des ÜK-CO₂ zum Zwecke der Extraktion von ausgetragenen Inhaltsstoffen aus dem ÜK-CO₂ oder ähnlichem in diesem Kreislauf sollte deshalb umgangen werden.

Bei dem Behandlungsgut handelt es sich in der Regel um ein poröses Material, aus dem sich die Luft nur schwer ganz verdrängen läßt. Um eine möglichst effektive Behandlung zu erreichen und dadurch lange und deshalb unwirtschaftliche Betriebszeiten zu vermeiden, ist es hilfreich den gefüllten Autoklaven vor der Behandlung mit ÜK-CO₂ mehrmals kurz zu evakuieren und mit Kohlendioxid zu spülen. Durch das Evakuieren vor der Behandlung wird im Anschluß das Material völlig mit unverdünnten ÜK-CO₂ durchdrungen.

Zur Behandlung äußerst resistenter Mikroorganismen kann durch eine Zugabe einer mikrobioziden Substanz die Behandlung unterstützt werden. Besonders wenn der Bedarf besteht nach der Behandlung einen dauerhaften Schutz des Schriftguts gegen einen erneuten Befall zu erhalten kann das Behandlungsgut über das überkritische Kohlendioxid mit einem Mikrobiozid (das für den Menschen keine Gefahr darstellen sollte) imprägniert werden.

Die Wirksamkeit der Behandlung mit ÜK-CO₂ ist in hohem Maße von der vorhandenen Feuchte sowie von der Temperatur, dem Druck und der Behandlungsdauer abhängig.

Durch Zusatz von Alkohol ist eine weitere Steigerung der Wirkung möglich. Durch Imprägnierung des Behandlungsgutes mit einem schwer flüchtigen Mikrobiozid (z.B. mit Captan (2-Trichlormethansulfenyl-(3ar, 7ac) - 3a, 4,7,7a - tetrahydro - isoindol - 1, 3 - dion)) mit Hilfe des ÜK-CO₂, ist es möglich dem Material einen dauerhaften Schutz vor einem mikrobiellen Befall zu verleihen.

Einzelne Anwendungsbeispiele sind im folgenden näher beschrieben. Alle in der vorliegenden Beschreibung genannten Prozentangaben sind als Gewichtsprozent zu verstehen.

### Beispiel 1:

Die zu behandelnden Papiere/Bücher stammen aus einem alten Unterbringungsgebäude und besitzen einen Feuchtigkeitsgehalt von 10 - 25%. Auf und in den Büchern befinden sich Schimmelpilze in der aktiven Wachstumsphase.

In diesem Fall werden die Bücher ohne Vorkonditionierung direkt in den Autoklaven gelegt und dort mit ÜK-CO₂ beaufschlagt. Vor der Behandlung mit ÜK-CO₂ wird der gefüllte Autoklav mehrmals kurz evakuiert und mit Kohlendioxid gespült.

Nach dem Schließen des Autoklaven kann der Druck in beliebiger Geschwindigkeit auf einen Wert oberhalb des kritischen Druckes von 72,8 Atmosphären angehoben werden. Der Arbeitsdruck beträgt mindestens 150 bar, vorzugsweise sogar über 250 bar.

In der Praxis wird der Maximaldruck verwendet, den die zur Verfügung stehende Anlage aufbringt, also meist bis zu 500 bar. Obergrenzen des Druckes, ab welchem Schädigungen am Schriftgut auftreten, sind bisher nicht bekannt.

Gleichzeitig mit dem Druck wird auch die Temperatur auf über 31,1°C, in der Regel auf 35°C oder 55°C, maximal jedoch 70°C - 110°C angehoben.

In einem Autoklaven wird, nach dem Einbringen der Bücher, dem Verschließen des Autoklaven und Spülen mit Kohlendioxid, in der Regel zunächst Kohlendioxid bei Umgebungsdruck und Umgebungstemperatur, also im gasförmigen Zustand, eingebracht und danach Druck und Temperatur in der vorbeschriebenen Weise gesteigert.

Die Haltezeit des überkritischen Zustandes im Autoklaven hängt einerseits vom Grad der mikrobiellen Kontaminierung der Bücher und andererseits von der Resistenz der Mikroorganismen im Behandlungsgut ab.

Je stärker die Ausgangskontamination ist, muß ein um so höherer Prozentsatz der Mikroorganismen durch die Behandlung zerstört werden, um mit den verbleibenden aktiven Spezies keine fortschreitende Zerstörung, sondern einen quasi stabilen Zustand des Buches in den künftigen Lagerungsbedingungen zu erzielen.

Die Mindesteinwirkungszeit liegt im Bereich von 30 - 90 Minuten, vorzugsweise, und um ausreichende Behandlungssicherheit zu erzielen, wird jedoch eine Behandlungszeit von 2 - 4 Stunden, in Extremfällen von bis zu 48 Stunden (in Einzelfällen bis zu einer Woche), gewählt.

Bei einem Behandlungsdruck von 200 Atmosphären und darüber sind Behandlungszeiten von 4 - 8 Stunden meist ausreichend.

### Beispiel 2:

Die Bücher besitzen einen Feuchtigkeitsgehalt von unter 8% bzw. der Feuchtigkeitsgehalt von über 8% der Bücher wurde nur kurzfristig herbeigeführt. Die Bücher sind mit Schimmel befallen, der allerdings hauptsächlich nur in der latenten Phase (überwiegend Schimmelpilze im Überdauerungszustand als Sporen) vorliegt.

In diesem Fall werden die Bücher konditioniert, indem sie einer feuchten Umgebung (z. B 25°C, 80% relativer Feuchtigkeit) über einen längeren Zeitraum ausgesetzt werden. Der Zeitraum muß nicht nur das Eindringen der Feuchtigkeit in das Innerste der Bücher gewährleisten, sondern dort auch über mindestens einen, vorzugsweise mindestens 3 Tage einwirken können, um vorhandene abgekapselte Mikroorganismen, insbesondere Pilzsporen, durch diese Feuchtigkeit "anzubrüten", also zu bewirken, daß diese Ihren physikalisch z. T. vielfach abgekapselten Zustand aufgeben und in einen aktiveren, geöffneten Zustand (Wachstumsphase) übergehen.

Anschließend wird das so vorkonditionierte Objekt in den Autoklaven zur Beaufschlagung mit ÜK-CO₂ verbracht, wobei die Zeit dazwischen nicht länger als 24 Stunden betragen sollte, um eine erneute Austrocknung und Abkapselung der Mikroorganismen zu vermeiden.

Bei der Vorkonditionierung ist wiederum darauf zu achten, daß keine Fließvorgänge der auf die Bücher einwirkenden Feuchtigkeit, also insbesondere kein Kondensieren in den Büchern, stattfindet.

In diesem Sinne sind also einerseits relevante Temperaturunterschiede zwischen Behandlungsobjekt und Behandlungsumgebung bei der Vorkonditionierung zu vermeiden, und ebenso Strömungsvorgänge an und in den Objekten.

Die Einwirkzeit bei der anschließenden Behandlung mit ÜK-CO₂ kann bei allen Beispielen durch erhöhte Temperatur auf maximal 50°C, insbesondere maximal 70°C bis 110°C verringert werden.

### Beispiel 3:

Für die Behandlung von Schriftgut, das durch sehr resistente Schimmelsporen im Überdauerungszustand belastet ist bietet sich die folgende Behandlung an:

Wenn es der Zustand des Schriftguts zuläßt sollten die Sporen in die aktive Wachstumsphase durch Konditionierung in einer feuchten Atmosphäre überführt werden wie in Beispiel 2.

Bei der anschließenden Behandlung mit ÜK-CO₂ wird zur Steigerung der Wirksamkeit zum ÜK-CO₂ 0,5 bis 2% Alkohol zugegeben. Hierfür eignen sich die leicht entfernbaren Alkohole Methanol, Ethanol, iso-Propanol, n-Propanol am besten. N-Propanol zeigte in dieser Reihe nach iso-Propanol die beste Wirksamkeit.

Die Behandlung sollte bei mind. 200 bar und mind. 55°C stattfinden und mindestens 4 Stunden dauern.

## Patentansprüche

1. Verfahren zur Inaktivierung mikrobieller Kontaminanten in Papieren, insbesondere in Form ganzer Bücher, wobei
die Papiere bzw. Bücher Kohlendioxid in überkritischem Zustand ausgesetzt werden,
**dadurch gekennzeichnet, daß**
die Papiere bzw. Bücher vor der Beaufschlagung mit überkritischem Kohlendioxid in feuchter Atmosphäre so vorkonditioniert werden, dass die resistenten Schimmelsporen vom inerten Überdauerungszustand in die empfindlichere aktive Wachstumsphase übergeführt werden.

2. Verfahren nach Anspruch 2,
**dadurch gekennzeichnet, daß**
die Papiere bzw. Bücher vor der Beaufschlagung mit überkritischem CO₂ in feuchter Atmosphäre, insbesondere von mindestens 60%, besser mindestens 75%, besser mindestens 80% relativer Luftfeuchtigkeit bei 20°C bis 40°C, vorkonditioniert werden, um resistente Schimmelsporen vom inerten Überdauerungszustand in die empfindlichere aktive Wachstumsphase zu überführen, insbesondere bis die Luftfeuchtigkeit in das Innerste der Papiere vorgedrungen ist, und insbesondere die Vorkonditionierung bei Normaldruck und mindestens 20°C über wenigstens 5 Stunden, insbesondere wenigstens 15 Stunden, insbesondere wenigstens 20 Stunden erfolgt, und insbesondere die Vorkonditionierung bei erhöhtem Druck von wenigstens 50 bar über wenigstens 3 Stunden, insbesondere wenigstens 8 Stunden insbesondere wenigstens 12 Stunden erfolgt.

3. Verfahren nach einem der vorhergehenden Ansprüche,
**dadurch gekennzeichnet, daß**
vor der Behandlung mit ÜK-CO₂ der für die Behandlung benutzte Autoklav möglichst mehrmals evakuiert und mit Kohlendioxid gespült wird.

4. Verfahren nach einem der vorhergehenden Ansprüche,
**dadurch gekennzeichnet, daß**
die Vorkonditionierung bis auf eine Feuchtigkeit des Papiers von mindestens 7%, insbesondere mindestens 10% erfolgt, und insbesondere zwischen der Vorkonditionierung und dem Beginn der Beaufschlagung mit überkritischem CO₂ eine Trocknung der Papiere bzw. Bücher auf unter 10%, insbesondere unter 7% Feuchtigkeit erfolgt und die Zeitdauer zwischen dem Ende der Vorkonditionierung und dem Beginn der Beaufschlagung mit überkritischem CO₂ maximal 8 Stunden, insbesondere 16 Stunden, insbesondere maximal 24 Stunden beträgt.

5. Verfahren nach einem der vorhergehenden Ansprüche,
**dadurch gekennzeichnet, daß**
zur Inaktivierung von hartnäckigen Mikroorganismen die Wirkung der Behandlung mit ÜK-CO₂ durch Erhöhung der Temperatur und/oder des Drucks und/oder durch die Verlängerung der Behandlungszeit gesteigert wird.

6. Verfahren nach einem der vorhergehenden Ansprüche,
**dadurch gekennzeichnet, daß**
durch Zugabe von 0,1 - 2,5% Alkohol (bezogen auf das ÜK-CO₂), insbesondere niedermolekulare, leicht flüchtiger Alkohole wie Ethanol besser n-Propanol, die Wirkung der Behandlung gesteigert und/oder die Behandlungszeit verkürzt wird, und insbesondere zwischen der Vorkonditionierung und dem Beginn der Beaufschlagung mit überkritischem CO₂ keine aktive Trocknung erfolgt und die Feuchtigkeit des Papiers bei Beginn der Beaufschlagung mit überkritischem CO₂ mindestens 5%, insbesondere mindestens 10% Feuchtigkeit beträgt und die Papiere bzw. Bücher nach der Behandlung mit überkritischem CO₂ auf unter 10%, insbesondere auf unter 7% Feuchtigkeit heruntergetrocknet werden.

7. Verfahren nach einem der vorhergehenden Ansprüche,
**dadurch gekennzeichnet, daß**
die Beaufschlagung mit überkritischem CO₂ ohne Anströmung der Papiere bzw. Bücher mit CO₂ erfolgt, insbesondere in einem abgeschlossenen Autoklaven, wobei das CO₂ so wenig wie möglich aktiv bewegt wird.

8. Verfahren nach einem der vorhergehenden Ansprüche,
**dadurch gekennzeichnet, daß**
am Ende der Beaufschlagung mit überkritischem CO₂ dessen Druck mit einer Geschwindigkeit von maximal 5 bar pro Minute auf Normaldruck abgesenkt wird, und insbesondere die Beaufschlagung mit überkritischem CO₂ bei mindestens 100 bar, insbesondere bei mindestens 200 bar, insbesondere bei mindestens 400 bar erfolgt, und insbesondere die Beaufschlagung mit überkritischem CO₂ bei mindestens 35°C, insbesondere mindestens 50°C erfolgt, und insbesondere die Beaufschlagung mit überkritischem CO₂ über mindestens 30 Minuten, vorzugsweise mindestens 4 Stunden erfolgt.

9. Verfahren nach einem der vorhergehenden Ansprüche,
**dadurch gekennzeichnet, daß**
bei der Inaktivierung ganzer Bücher die Beaufschlagung mit überkritischem CO₂, insbesondere auch die Vorkonditionierung, an den ganzen, geschlossenen Büchern erfolgt.

10. Verfahren nach einem der vorhergehenden Ansprüche,
**dadurch gekennzeichnet, daß**
zur Inaktivierung von durchnäßten Papieren bzw. Büchern die Beaufschlagung mit überkritischem CO₂ vor der Trocknung, die insbesondere mittels Tiefkühlen erfolgt, geschieht.

11. Verfahren nach einem der vorhergehenden Ansprüche,
**dadurch gekennzeichnet, daß**
die Beaufschlagungszeit mit überkritischem CO₂ um so länger gewählt wird, je niedriger der Feuchtigkeitsgehalt der Papiere bzw. Bücher d.h. je resistenter die Mikroorganismen sind (beispielsweise Sporen im Überdauerungszustand).

12. Verfahren nach einem der vorhergehenden Ansprüche,
**dadurch gekennzeichnet, daß**
dem überkritischen CO₂ ein Imprägniermittel evtl. auch in Kombination mit einem Kosolvent (beispielsweise Alkohole oder Alkane), insbesondere zur Verbesserung der mechanischen Festigkeit der Papiere, zugesetzt ist, welches wenigstens teilweise im überkritischen Kohlendioxid gelöst wird.

13. Verfahren nach einem der vorhergehenden Ansprüche
**dadurch gekennzeichnet, daß**
dem überkritischen CO₂ ein Mikrobiozid zugesetzt ist (auch in Kombination eines weiteren Lösungsmittels), welches wenigstens teilweise im überkritischen Kohlendioxid gelöst wird.

## Claims

1. A process for the deactivation of microbial contaminants in paper, in particular in the form of complete books, in which
the paper or books are exposed to carbon dioxide in a supercritical state,
**characterized in that**,
before application of supercritical carbon dioxide in a humid atmosphere, the papers or books are pre-conditioned so that the resistant mold spores are converted from the inert survival state to the more susceptible active growth phase.

2. A process as set forth in claim 2,
**characterized in that**,
the paper or books are pre-conditioned in a more humid atmosphere before application with supercritical CO₂, in particular, with at least 60%, preferably 75% and more preferably 80% relative humidity at 20° to 40°C, in order to convert the resistant mold spores from the inert survival state to the more susceptible active growth phase, particularly until the humidity has penetrated into the innermost papers and in particular that the pre-conditioning takes place at normal pressure and at least 20° for at least 5 hours, preferably at least 15 hours, in particular at least 20 hours, and preferably that pre-conditioning occurs at increased pressure of at least 50 bars for at least 3 hours, preferably at least 8 hours and more preferably at least 12 hours.

3. Process under one of the foregoing claims,
**characterized in that**,
before treatment with the supercritical CO₂, the autoclave used for treatment is preferably evacuated a plurality of times and flushed with carbon dioxide.

4. A process as set forth in one of the foregoing claims,
**characterized in that**,
the pre-conditioning occurs up to a moisture content of the paper of at least 7%, preferably at least 10%, and in particular between the pre-conditioning and the start of application with supercritical CO₂, the paper or books is dried to under 10%, preferably under 7% moisture content and the period between the end of pre-conditioning and the start of application with supercritical CO₂ equals at most 8 hours, preferably a maximum of 16 hours and more particularly a maximum of 24 hours.

5. A process as set forth in one of the foregoing claims,
**characterized in that**,
for deactivation of persistent microorganisms, the affect of treatment with supercritical CO₂ is augmented through an increase in the temperature and/or the pressure and/or through a lengthening of the treatment time.

6. A process as set forth in one of the foregoing claims,
**characterized in that**,
through the addition of 0.1 -2.5% alcohol (relative to the supercritical CO₂), preferably low molecular, easily volatile alcohols such as ethanol, or preferably n-propanol, the affect of treatment is augmented and/or the treatment time is shortened, and in particularly no active drying takes place between the pre-conditioning and the start of the application of CO₂ and the moisture content of the paper at the start of application of CO₂ is at least 5% and preferably 10% and after treatment with supercritical CO₂ the paper or books are dried to a moisture content below 10%, preferably below 7%.

7. A process as set forth in one of the foregoing claims,
**characterized in that**,
treatment with supercritical carbon dioxide is effected without the carbon dioxide being in flow relationship with the item, the CO₂ being actively moved as little as possible.

8. A process as set forth in one of the foregoing claims,
**characterized in that**,
at the end of the treatment with supercritical CO₂ the pressure thereof is reduced to normal pressure at a rate of a maximum of 5 bars per minute, and in particular the treatment with supercritical CO₂ is effected at a pressure of at least 100 bars, preferably at least 200 bars, more preferably at least 400 bars, and in particular the treatment with supercritical CO₂ is effected at at least 35°C, preferably at at least 50°C, and in particular the treatment with supercritical CO₂ is effected over at least 30 minutes, preferably over at least 40 hours.

9. A process as set forth in one of the foregoing claims,
**characterized in that**,
the item to be inactivated is whole books and the treatment with supercritical CO₂, preferably also the pre-conditioning, is effected on the whole books in the closed condition.

10. A process as set forth in one of the foregoing claims,
**characterized in that**,
for the purpose of inactivating soaked paper the treatment with supercritical CO₂ is effected prior to an operation of drying the paper, which preferably is effected by means of freezing.

11. A process as set forth in one of the foregoing claims,
**characterized in that**,
the duration of the treatment with supercritical CO₂ is increased in proportion to a lower moisture content of the paper or books and thus greater resistance of the microorganisms (such as spores in the survival condition).

12. A process as set forth in one of the foregoing claims,
**characterized in that**,
an impregnation agent is added to the supercritical carbon dioxide, optionally also in combination with a co-solvent, in particular to improve the mechanical strength of paper, which is at least partially dissolved in the supercritical CO₂.

13. A process as set forth in one of the foregoing claims,
**characterized in that**,
a microbiociodal agent is added to the supercritical carbon dioxide (also in combination with a further solvent) said agent being at least partially dissolved in the supercritical CO₂.

## Revendications

1. Procédé pour l'inactivation de contaminants microbiens dans le papier en particulier sous forme de livres entiers,
les papiers respectivement les livres étant exposés au dioxyde de carbone dans un état hypercritique.
**caractérisé en ce que** les papiers respectivement les livres ont été préconditionnés avant l'impact du CO₂ hypercritique dans une atmosphère humide de sorte que les pores de moisissures résistants soient amenés de l'état inerte de survie à la phase de croissance active plus sensible.

2. Procédé selon la revendication 2, **caractérisé en ce que** les papiers respectivement les livres ont été préconditionnés avant l'impact du dioxyde de carbone hypercritique dans une atmosphère humide en particulier d'au moins 60 % de préférence d'au moins 75 %, idéalement d'au moins 80 % d'humidité relative de l'air à une température comprise entre 20°C et 40°C pour amener les pores de moisissures résistants de l'état inerte de survie à la phase de croissance active plus sensible, en particulier jusqu'à ce que l'humidité de l'air ai pénétré au coeur du papier et en particulier le préconditionnement s'effectue à pression normale et au moins à 20°C pendant moins 5 heures, en particulier pendant au moins 20 heures et en particulier le préconditionnement s'effectue à une pression plus élevée d'au moins 50 bar au moins pendant 3 heures en particulier au moins pendant 8 heures, en particulier au moins 12 heures.

3. Procédé selon l'une des revendications précédentes, **caractérisé en ce qu'**avant le traitement avec le CO2 hypercritique l'autoclave, utilisé pour le traitement est évacué dans la mesure du possible plusieurs fois et est balayé au dioxyde de carbone.

4. Procédé selon l'une des revendications précédentes, **caractérisé en ce que** le préconditionnement s'effectue jusqu'à une humidité du papier d'au moins 7 % en particulier au moins 10 %, et en particulier entre le préconditionnement et le début de l'impact avec le CO₂ hypercritique, un séchage des papiers respectivement des livres s'effectue à moins de 10%, en particulier à moins de 7 % d'humidité et la durée entre la fin du préconditionnement et le début de l'impact avec du CO₂ hypercritique est de maximum 8 heures, en particulier 16 heures, en particulier maximum 24 heures.

5. Procédé selon l'une des revendications précédentes, **caractérisé en ce que** pour l'inactivation de microorganismes tenaces, l'action du traitement au CO₂ hypercritique par augmentation de la température et/ou de la pression et/ou par prolongement du temps de traitement est augmentée.

6. Procédé selon l'une des revendications précédentes, **caractérisé en ce que** par ajout de 0,1 à 2,5 % d'alcool (par rapport au CO₂ hypercritique), en particulier des alcools volatiles de basse molécularité tels que l'éthanol, de préférence le n-propanol, l'action du traitement est augmentée et/ou le temps de traitement est réduit et en particulier entre le préconditionnement et le début de l'impact avec du CO₂ hyperactif, il ne s'effectue aucun séchage actif et l'humidité du papier au début de l'impact avec du CO₂ hypercritique est d'au moins 5 % en particulier au moins 10 % et les papiers respectivement les livres sont séchés après le traitement avec le CO₂ hypercritique est amené à moins de 10% en particulier à moins de 7 % 'humidité.

7. Procédé selon l'une des revendications précédentes, **caractérisé en ce que** l'impact avec du CO₂ hypercritique s'effectue sans écoulement du CO₂ sur les papiers respectivement les livres, en particulier dans un autoclave fermé, le CO₂ étant déplacé activement le moins possible.

8. Procédé selon l'une des revendications précédentes, **caractérisé en ce qu'**à la fin de l'impact de CO₂ hypercritique dont la pression est abaissée à une vitesse de maximum 5 bars par minute à la pression normale et en particulier l'impact avec le CO₂ hypercritique s'effectue à au moins 100 bars, en particulier au moins 200 bars, en particulier an moins 400 bars et en particulier l'impact au CO₂ hypercritique s'effectue à au moins 35°C en particulier au moins 50° et en particulier l'impact au CO₂ hypercritique s'effectue pendant au moins 30 minutes, de préférence au moins 4 heures.

9. Procédé selon l'une des revendications précédentes, **caractérisé en ce que** lors de l'inactivation de livres entiers, l'impact au CO₂ hypercritique en particulier le préconditionnement s'effectuent sur les livre entiers fermés.

10. Procédé selon l'une des revendications précédentes, **caractérisé en ce que** pour l'inactivation de papiers respectivement de livres mouillés, l'impact au CO₂ hypercritique se produit avant le séchage qui s'effectue en particulier par refroidissement profond.

11. Procédé selon l'une des revendications précédentes, **caractérisé en ce que** plus la teneur en humidité des papiers respectivement des livres est basse c'est-à-dire plus les microorganismes sont résistants (par exemple des pores à l'état de survie) et plus le temps d'impact au CO₂ hypercritique est sélectionné long.

12. Procédé selon l'une des revendications précédentes, **caractérisé en ce qu'**il est ajouté au CO₂ hypercritique un moyen d'imprégnation éventuellement en combinaison avec un cosolvant (en particulier des alcools ou des alcanes), en particulier pour l'amélioration de la résistance mécanique des papiers, moyen qui se dissout au moins partiellement dans le dioxyde de carbone hypercritique.

13. Procédé selon l'une des revendications précédentes, **caractérisé en ce qu'**il est ajouté au CO₂ hypercritique un microbiocide (également en combinaison avec un autre solvant), qui se dissout au moins partiellement dans le CO₂ hypercritique
